# EUROPEAN PATENT SPECIFICATION

(11) **EP 3 530 230 B1**
(45) Date of publication and mention of the grant of the patent: **06.04.2022**
(21) Application number: 19157829.3
(22) Date of filing: 18.02.2019
(51) Int. Cl.: A61C 7/08, A61F 5/56

(54) **ORAL DEVICE FOR MYOFUNCTIONAL RE-EDUCATION**
ORALE VORRICHTUNG ZUM ERNEUTEN ERLERNEN DER MYOFUNKTION
DISPOSITIF ORAL POUR LA RÉÉDUCATION MYOFONCTIONNELLE

(30) Priority: 27.02.2018 IT 201800003076
(43) Date of publication of application: 28.08.2019
(73) Proprietor: Sintucci, Fabrizio, 47522 Cesena (FC) (IT)
(72) Inventor: Sintucci, Fabrizio, 47522 Cesena (FC) (IT)
(74) Representative: Busca, Andrea

(56) References cited:
- WO-A1-2017/020079
- DE-C- 65 194
- FR-A1- 2 867 058
- FR-A1- 2 930 138
- US-A- 4 304 227

## Description

The present invention relates to an oral device for myofunctional re-education.

The invention was made with particular reference to the treatment of atypical swallowing.

Atypical swallowing is physiological in children during lactation, but when it persists also after they have teeth it generates various problems, including: open bite or crossbite in teething; oral respiration; nighttime snoring, reaching apnoea, recurrent tonsillitis, adenoid hypertrophy, chronic otitis, reduced hearing in the child.

The typical treatment for atypical swallowing is handled by a speech and language therapist, who implements a series of neuromuscular rebalancing exercises, particularly of the tongue. In a first step the exercises are of the conscious type and in a second step it is necessary to stabilise the results obtained by making the new swallowing posture unconscious.

However, performing the exercises is a demanding practice, especially when the subject being treated is a child.

Another problem with traditional speech and language therapist treatment comes from the fact that the child's attention is placed on the apical part of the tongue, which is lifted in the search for the retroincisive papilla. However, this action triggers a lowering of the rear part of the tongue or, better, an obstruction to the action of the styloglossus muscle which causes it to rise. The correct physiological position, on the contrary, envisages the rear part of the tongue being raised and preferably in contact with the palate.

As an alternative to therapy through exercises, orthodontic devices are known that act as a shield between the tongue and the teeth. They are, for example, grilles, tongue spurs, mandible compression plates, Tucat's pearls, etc.

It is clear that such devices are very invasive, especially for children, as well as not guaranteeing definitive re-education.

In patent literature, an oral device is also known from WO2017/020079, but it is unsuitable as rather than inducing correct nasal respiration, it simulates the latter through oral respiration thanks to a special passage of air through a bite.

Oral devices are also known in patent literature from DE65194C, US4304227, FR2930138 and FR2867058.

The first is unsuitable for the purpose as flanges external to the lips and internal to the oral cavity obstruct the natural posture of the mouth; notably worsening the posture there is also a flange that presses the tongue downwards.

The last two patent documents propose a tongue suction channel between the lips, which however worsens the situation as it distances the rear portion of the tongue from the palate. Furthermore, such position perpetrated for a long time, especially in a child, hinders the enlargement of the palate and the consequent correct descent of the teeth.

US 4 304 227 A discloses a device for treatment of snoring having support surfaces for lower and upper lips, the support surfaces are shaped to form a continuous annular contact surface with the lips; projecting walls forming a continuous annular collar around said annular contact surface; wherein the annular contact surface and the annular collar are connected to each other in an airtight way along the whole annular perimeter, e.g. with structural continuity.The oral device further comprises a portion that obstructs the passage between the lips placed inside the zone surrounded by the annular contact surface for sealing the passage of air and of the tongue. An object of the present invention is to overcome all or some of the drawbacks of the prior art.

A preferred object of the present invention is that of providing an oral device that can be worn at any time to provide unconscious speech and language therapy re-education of the mouth, especially of the tongue.

A further preferred object of the present invention is that of providing a device suitable for children to promote the correct formation of the mouth, in particular the enlargement of the palate and the consequent correct descent of the teeth.

A further preferred object of the present invention is that of providing a device that is simple to use and not invasive.

Another further preferred object of the present invention is that of providing a device that is particularly ergonomic and comfortable to wear.

Another preferred object of the present invention is that of providing a device that is easy and cheap to produce.

The scope of the invention is defined by the appended claims.

Advantageously, the device of the present invention can be worn during any activity and therefore it trains the patient on an unconscious posture of the mouth, in particular the device keeps the lips apart promoting the raising of the rear part of the tongue. Furthermore, the obstruction is particularly important in preventing oral respiration and in promoting nasal respiration. The device further prevents the suction of the tongue and therefore promotes correct contact with the palate.

It has been found that all this leads to notable resolutive results of atypical suction.

Preferably, the obstruction portion has structural bearing characteristics of said lip support surfaces such as to oppose their crushing between the lips.

With reference to a situation in which the device is clamped between the lips, and considering a general convention of the present invention in which radial direction is intended to mean the distancing and nearing direction of the lips to/from each other and axial direction is intended to mean the entry and exit direction from the mouth, orthogonal to the radial direction, particularly ergonomic embodiments that are comfortable to wear are envisaged wherein the projecting walls extend in a radial or substantially radial direction, and the support surfaces extend in the axial and/or substantially axial direction and preferably according to connecting portions to the projecting walls.

According to some preferred embodiments, the oral device comprises a bite type device joined to the lip support surfaces, the bite device being free from suction channels of the tongue through it.

Preferably, said obstruction portion comprises at least one joining portion to said bite device.

The device comprises at least one support ramp (260) for supporting the tongue with an inclination towards the palatine spot for resting below the tongue and bearing it. The inclination is ascending from the tongue attachment base towards the free tip of the tongue. This guides the tip of the tongue towards said palatine spot, being a desirable posture, but unlike traditional speech and language therapist treatment the support surfaces that keep the lips dilated generate as a reflection a raising of the rear part of the tongue, solving the problem of the prior art.

Preferably, said bite device comprises a support wall for supporting the dental arches clamped between them, said wall being free from passages for the tongue through it. For example, the support wall acts as a support for both of the dental arches and is a full wall, i.e. not hollow.

According to a preferable general characteristic, the support surfaces are arch or substantially arch shaped, so as to have a maximum distance between them at the centre of the arches, where the distance, or the minimal distance, between the centres of the arches is greater than 1.5 cm. This is particularly useful in providing an optimal stimulus to the raising of the rear part of the tongue.

According to some embodiments that are particularly easy and cheap to produce, the device is entirely made of elastic material, e.g. made as a single block, e.g. by moulding.

According to some preferred embodiments of the invention, the support surfaces are arch shaped and are joined at the ends, preferably substantially forming an ellipse. To further increase the ergonomics, the arches can be concave, in the radial direction, but also in the axial direction.

According to a general preferable characteristic of the invention, the device is free from walls projecting from the support surfaces and intended to be positioned outside the mouth in a position facing the projecting walls intended to be interposed between the lips and the dental arches. In this way, the lips are free towards the outside, so that the device is more comfortable to wear and facilitates labial exercise, promoting the unconscious learning of the posture.

According to some preferred embodiments, the support surfaces extend in an arch and comprise first portions 5a and 10a that extend in the axial and/or substantially axial direction and second connecting portions to the projecting walls, so as to allow surface continuity therewith, where:
- the first portion of the upper support surface has an extension in the axial or substantially axial direction that becomes thinner from the ends towards the centre of the related arch,
- on the contrary, the second portion of the lower support surface has an extension in the axial or substantially axial direction that becomes thicker from the ends towards the centre of the related arch.

This promotes ergonomic contact with the lips that hinders the passage of air.

Further characteristics and advantages of the present invention will become clearer from the following detailed description of the preferred embodiments thereof, with reference to the appended drawings and provided by way of indicative and non-limiting example. In such drawings:

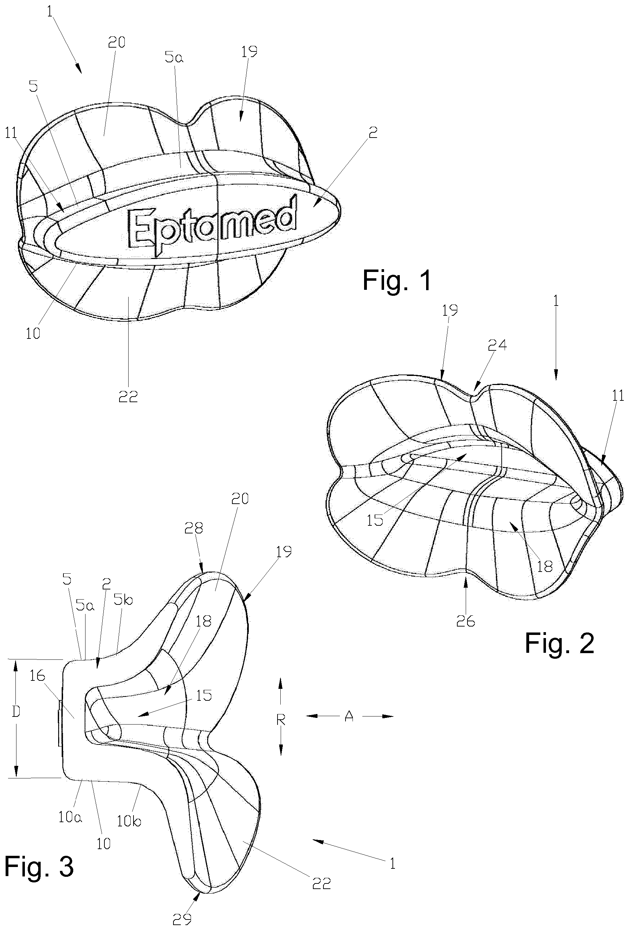

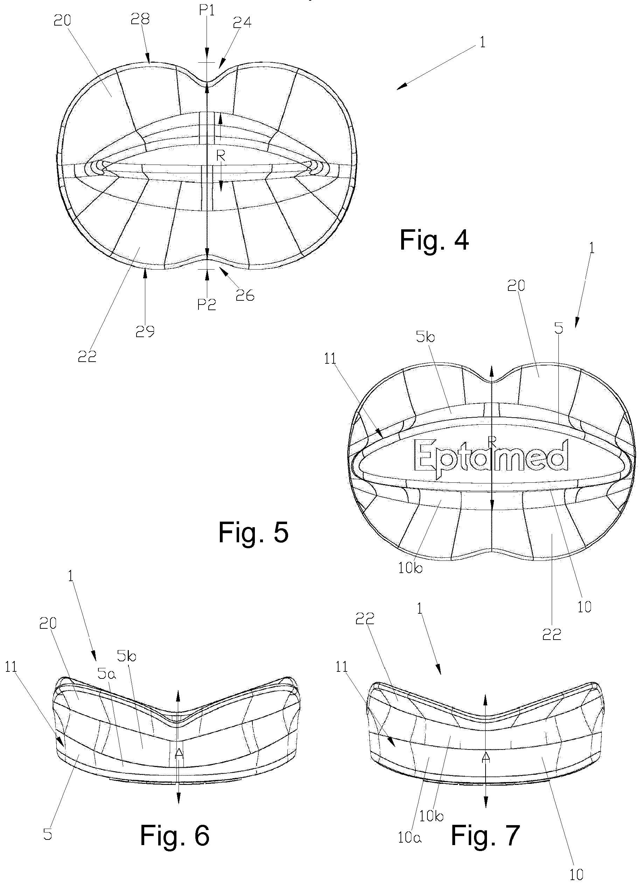

- figures 1 to 7 schematically represent an example not covered by the invention , in particular, with reference by convention to the device clamped between the lips of a patient in the erect position, Figure 1 is a perspective view from an external observation point to the mouth, Figure 2 is a perspective view from an internal observation point to the mouth, Figure 3 is a lateral sectional view, Figure 4 is a rear view from an internal observation point to the mouth, Figure 5 is a front view from an external observation point to the mouth, Figure 6 is a view from above, Figure 7 is a view from below;
- Figures 8 to 10 represent some views like the previous ones of an alternative embodiment not covered by the present invention;
- Figures 11 to 16 represent some views like the previous ones of an embodiment according to the present invention comprising a bite type device joined in tandem with the parts of the previous figures;
Figure 17 schematically represents the device of figures 1 to 7 worn by a user to show the posture of the tongue.

With reference to figure 1 an oral device is shown adapted to perform myofunctional re-education according to the present invention and indicated overall with the reference number 1.

Oral device means a device adapted to be worn in the mouth, speech and language therapist re-education means the re-education of one or more parts of the mouth, such as the tongue, for a correct swallowing position.

In the present description, the following definitions are used conventionally with reference to the device clamped between the lips:
- radial direction: the distancing and nearing direction of the lips from/to each other, indicated in Figures 3, 4 and 5 with the arrows R;
- axial direction: the entry and exit direction from the mouth, orthogonal to the radial direction, indicated with the arrows A in Figures 3, 6 and 7.

The device 1 comprises a base body 2, adapted to be interposed between the lips, radially delimited by a support surface 5 for the upper lip and by a support surface 10 for the lower lip.

Each support surface extends according to an arch, where the two arches are joined to the ends so as to have the maximum distance at the centre. The resulting figure is an annular support surface, substantially an ellipse.

The minimum distance D between the centres of the arches 5 and 10 is greater than 1.5 cm.

The arches are preferably concave both in the radial direction R, to keep the lips apart, and in the axial direction A (Figures 6 and 7), for conforming to the anatomy of the lips.

In general it is preferable that the two arches are different from each other, in particular that the arch of the lower support surface 10 is pressed more in the radial direction with respect to the arch of the upper support surface 5, as can be seen better in Figure 5.

The area surrounded by the arches 5 and 10 is closed in an airtight way, in particular between such arches there is an obstruction portion 16 of a cavity 15 with a blind bottom present in the base body 2. The cavity 15 has an opening 18 on the opposite side, in the axial direction A, to the obstruction portion 16.

From the base body 2, in an area intended to be placed inside the mouth, a pair of projecting walls 20, 22 branch off in the radial R or substantially radial direction. Such directions are to be considered in the wide sense and with the benefit of interpretation, as they have to adapt to the anatomical requirements of the mouth.

A wall 20 is adapted to be interposed between the upper lip and the upper dental arch when the support surfaces are clamped between the lips; and the other wall 22 is adapted to be interposed between the upper lip and the upper dental arch when the support surfaces are clamped between the lips.

The two walls 20, 22 are joined together to form an annular collar 19 around the base body 2. The annular collar is continuous around said annular contact surface; furthermore, the annular contact surface 11 and the annular collar 19 are connected to each other in an airtight way along the entire annular perimeter, e.g. with structural continuity. This means that between the annular contact surface 11 and the annular collar 19 there are no air passages.

The outer edges 28 and 29 of the projecting walls 20, 22 are shaped at least to house the labial frenula, e.g. at the centre they have respective recesses 24, 26 in the radial direction with respective depths P1 and P2 such for which P1 >=2.5 mm ??? and P2 >= 1.4 mm.

As can be seen better in the section of Figure 3, the projecting walls 20 and 22 preferably have a thickness that becomes narrower from the base body 2 towards the outer edges 28 and 29.

It is observed that the support surfaces 5 and 10 preferably comprise first portions 5a and 10a that extend in the axial and/or substantially axial direction A and second connecting portions 5b and 10b to the projecting walls 20, 22, so as to allow surface continuity therewith.

The first portion 5a of the upper support surface 5 preferably has an extension in the axial or substantially axial direction that becomes thinner from the ends towards the centre of the related arch, as is clearly visible in Figure 6.

On the contrary, the second portion 10a of the lower support surface 10 preferably has an extension in the axial or substantially axial direction that becomes thinner from the ends towards the centre of the related arch, as is clearly visible in Figure 7.

The device is free from walls intended to be positioned outside the mouth facing the projecting walls 20 and 22. In this way the lips are not contained between two walls, being free towards the outside of the mouth.

The device preferably comprises an elastic material, e.g. an elastomer. More preferably, the device 1 is a single block of such material.

Some alternative examples not covered by the invention are described below where same or similar elements are indicated with the same reference numbers used above, or with the same numbers increased by 100 or multiples thereof.

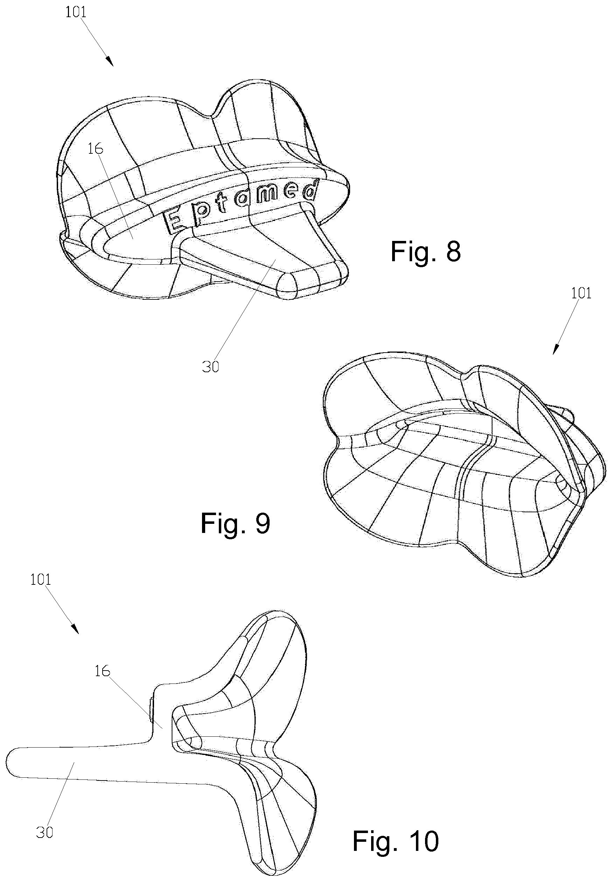

With reference to Figures 8 to 10, an example not covered by the invention is shown, indicated overall with number 101 and that differs from the previous one at least in that it comprises a gripping portion 30, projecting from the base body 2 outside the patient's mouth. Preferably the projecting portion 30 projects from the obstruction portion 16, e.g. in the axial direction A. Preferably it is a rod. However, other forms are not excluded such as, for example, dummy-type gripping handles, rings, etc.

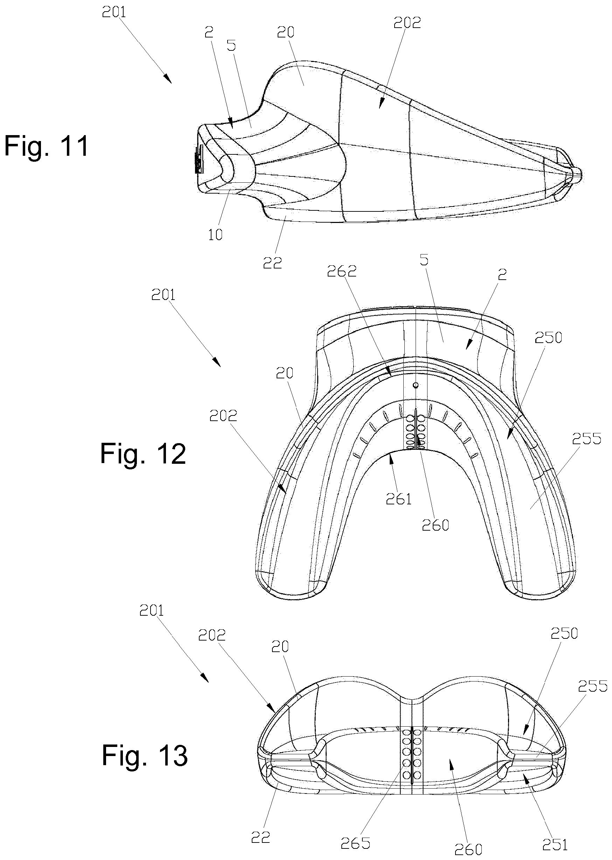

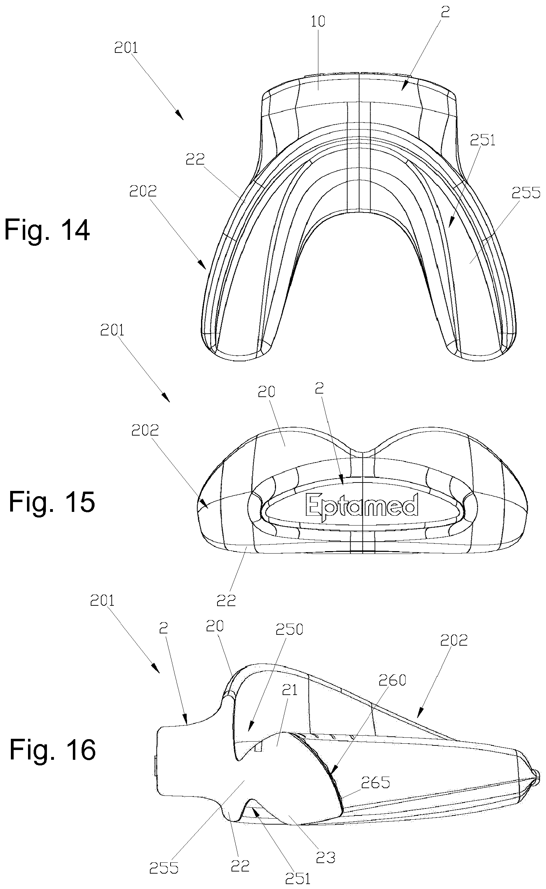

With reference to Figures 11 to 16, an embodiment according to the invention is shown, indicated overall with number 201 and that differs from the previous ones at least in that it comprises a bite type device 202 intended to be interposed between the dental arches. In particular, the bite type device is joined to the base portion 2 and projects into the mouth.

A bite device is traditionally a device adapted to act on dental arches as a re-balancer/activator.

The bite type device 202 comprises at least one seat 250 for at least partially receiving a dental arch, e.g. the upper arch.

The seat 250 comprises at least one bottom wall 255 intended to be interposed between the dental arches and at least one wall 20 projecting radially or substantially radially from said bottom wall for delimiting the seat at least on one side. For example such wall can coincide with one of the walls 20 projecting from the lip support surfaces 5, 10. In the preferred embodiments the seat 250 is preferably in the form of a channel delimited by the bottom wall 255 and by two lateral walls 20, 21 adapted to receive, interposed between them, the dental arch.

The bite type device 202 has at least a second seat 251 for at least partially receiving the other dental arch, e.g. the lower dental arch.

The seat 251 comprises at least one bottom wall 255 intended to be interposed between the dental arches, preferably coinciding with that of the seat 250, so as to form a single element therewith. The seat 251 also comprises at least one wall 22 projecting radially or substantially radially from said bottom wall for delimiting the seat at least on one side. For example such wall can coincide with one of the walls 22 projecting from the lip support surfaces 5, 10. In the preferred embodiments the seat 251 is preferably in the form of a channel delimited by the bottom wall 255 and by two lateral walls 22, 23 adapted to receive, interposed between them, the dental arch.

The bite type device 202 further comprises a ramp 260 for the support of the tongue. It is fashioned, for example, on the rear part of one of the receiving seats 250, 251, so as to be joined through it to the base portion 2.

The ramp 260 is inclined to guide the tip of the tongue towards the palatine spot, therefore it ascends from a proximal end 261, to a distal end 262. Preferably the ramp 260 comprises at least one protuberance and/or recess 265 for stimulating the tongue.

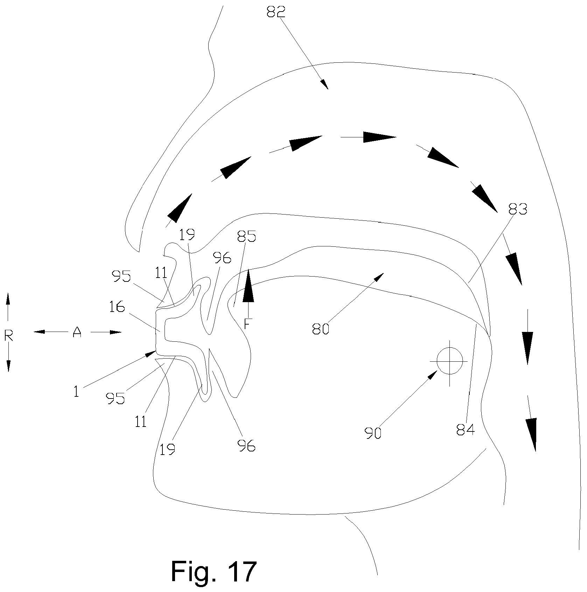

Figure 17 shows the effect on the posture of a patient's tongue of the device 1 of Figures 1 to 7. The same effect can be found with the other devices described in the subsequent figures.

In particular the skull of a user with the oral cavity 80 and the nasal cavity 82 is observed in a sectional view. In the oral cavity 82 the rear part of the palate 84 and of the tongue 83 are visible, which touch each other when the device 1 is worn with the annular support collar 11 between the lips 95 and the annular collar 19 between the lips 95 and the teeth 96. The posture of the tongue is enabled because its rear musculature 90 is not contracted by a suction forwards. In fact, the blind cavity 15 is generally unsuitable in terms of width and depth for sucking the tongue, and may be absent. The tip 85 of the tongue 84, unlike what happens in devices with a suction channel, is raised (arrow F) and is positioned naturally at the palatine spot. All this induces nasal respiration, as indicated by the arrows in the nasal cavity 82.

### GENERAL INTERPRETATION OF TERMS

In understanding the object of the present invention, the term "comprising" and its derivatives, as used herein, are intended as open-ended terms that specify the presence of declared characteristics, elements, components, groups, integers and/or steps, but do not exclude the presence of other undeclared characteristics, elements, components, groups, integers and/or steps. The above also applies to words that have similar meanings such as the terms "comprised", "have" and their derivatives. Furthermore, the terms "part", "section", "portion", "member" or "element" when used in the singular can have the double meaning of a single part or a plurality of parts. As used herein to describe the above executive embodiment(s), the following directional terms "forward", "backward", "above", "under", "vertical", "horizontal", "below" and "transverse", as well as any other similar directional term, refers to the embodiment described in the operating position. Finally, terms of degree such as "mainly", "about" and "approximately" as used herein are intended as a reasonable amount of deviation of the modified term such that the final result is not significantly changed.

## Claims

1. Myofunctional Oral device for myofunctional re-education adapted to be worn between a patient's lips, comprises:
- a support surface for the upper lip (5),
- a support surface for the lower lip (10),
- a wall (20) projecting from the upper support surface (5) in a position adapted to be interposed between the upper lip and the upper dental arch when the support surfaces are clamped between the lips;
- a wall (22) projecting from the lower support surface (10) in a position adapted to be interposed between the lower lip and the lower dental arch when the support surfaces are clamped between the lips;
**characterised in that**
- the support surfaces are shaped to form a continuous annular contact surface (11) with the lips;
- the projecting walls form a continuous annular collar (19) around said annular contact surface (11);
- the annular contact surface (11) and the annular collar (19) are connected to each other in an airtight way along the whole annular perimeter, e.g. with structural continuity;
- the myofunctional oral device comprises a portion (16) that obstructs the passage between the lips placed inside the zone surrounded by the annular contact surface (11) for sealing the passage of air and of the tongue, wherein
the myofunctional oral device comprises a bite type device (202) joined to the lip (11) support surfaces, the bite device being free from suction channels of the tongue through it
wherein said obstruction portion comprising at least one joining portion to said bite device
and wherein said bite device comprises at least one support ramp (260) for supporting the tongue with an inclination towards the palatine spot for resting below the tongue and bearing it.

2. Device according to claim 1, **characterised in that** the obstruction portion (16) has structural bearing characteristics of said lip (5, 10) support surfaces such as to oppose their crushing between the lips.

3. Device according to any one of the previous claims, **characterised in that** with reference to a situation in which the device is clamped between the lips (95) and conventionally considering that radial direction (R) means the distancing and nearing direction of the lips from/to each other and axial direction (A) means the entry and exit direction from the mouth orthogonal to the radial direction, the projecting walls (20, 22) extend in the radial (R) or substantially radial direction, the support surfaces extend in the axial (A) and/or substantially axial direction and preferably according to connection portions (5b, 10b) to the projecting walls.

4. Device according to any one of the preceding claims, **characterised in that** said bite device comprises a support wall for supporting the dental arches clamped between them, said wall being free from passages for the tongue through it.

5. Device according to any one of the previous claims, **characterised in that** the support surfaces (5, 10) are arch or substantially arch shaped, so as to have a maximum distance (D) between them at the centre of the arches, where the distance (D), or the minimal distance, between the centres of the arches is preferably greater than 1.5 cm.

6. Device according to any one of the previous claims, **characterised in that** it is entirely made of elastic material.

7. Device according to any one of the previous claims, **characterised in that** it is free from walls projecting from the support surfaces (5, 10) and intended to be positioned outside the mouth in a position facing the projecting walls (20, 22) intended to be interposed between the lips and the dental arches.

8. Device according to any one of the previous claims, **characterised in that** it comprises a gripping portion (30), preferably a rod, adapted to project outside the mouth.

9. Device according to any one of the preceding claims, **characterised in that** the support surfaces (5, 10) extend in an arch and comprise first portions 5a and 10a that extend in the axial and/or substantially axial direction (A) and second connecting portions (5b, 10b) to the projecting walls (20, 22), so as to allow surface continuity therewith, where:
- the first portion (5a) of the upper support surface (5) has an extension in the axial or substantially axial direction that becomes thinner from the ends towards the centre of the related arch,
- on the contrary, the second portion (10a) of the lower support surface (10) has an extension in the axial or substantially axial direction that becomes thicker from the ends towards the centre of the related arch.

## Patentansprüche

1. Myofunktionelle orale Vorrichtung zur myofunktionellen Umerziehung, die dazu geeignet ist, zwischen den Lippen eines Patienten getragen zu werden, umfasst:
- eine Auflagefläche für die Oberlippe (5),
- eine Auflagefläche für die Unterlippe (10),
- eine Wand (20), die von der oberen Auflagefläche (5) in einer Position vorsteht, die geeignet ist, zwischen der Oberlippe und dem oberen Zahnbogen eingefügt zu werden, wenn die Auflageflächen zwischen den Lippen eingeklemmt sind;
- eine Wand (22), die von der unteren Auflagefläche (10) in einer Position vorsteht, die geeignet ist, zwischen der Unterlippe und dem unteren Zahnbogen eingefügt zu werden, wenn die Auflageflächen zwischen den Lippen eingeklemmt sind;
**dadurch gekennzeichnet, dass**
- die Auflageflächen sind so geformt, dass sie eine durchgehende ringförmige Kontaktfläche (11) mit den Lippen bilden;
- die vorstehenden Wände einen durchgehenden ringförmigen Kragen (19) um die ringförmige Kontaktfläche (11) bilden;
- die ringförmige Kontaktfläche (11) und der ringförmige Kragen (19) entlang des gesamten ringförmigen Umfangs luftdicht miteinander verbunden, z. B. mit struktureller Kontinuität;
- die myofunktionelle orale Vorrichtung einen Abschnitt (16) umfasst, der den Durchgang zwischen den Lippen versperrt, der sich innerhalb der von der ringförmigen Kontaktfläche (11) umgebenen Zone befindet, um den Durchgang der Luft und der Zunge abzudichten, **dadurch gekennzeichnet, dass**
die myofunktionelle orale Vorrichtung eine Vorrichtung vom Typ einer Aufbissschiene (202) umfasst, die mit den Auflageflächen der Lippe (11) verbunden ist, wobei die Aufbissschienenvorrichtung frei von Saugkanälen der Zunge durch sie ist
wobei der Hindernisabschnitt mindestens einen Verbindungsabschnitt zu der Aufbissschienenvorrichtung umfasst
und wobei die Aufbissschienenvorrichtung mindestens eine Stützrampe (260) zum Stützen der Zunge mit einer Neigung zur Gaumenstelle hin aufweist, um unter der Zunge zu ruhen und sie zu tragen.

2. Vorrichtung nach Anspruch 1, **dadurch gekennzeichnet, dass** der Hindernisabschnitt (16) strukturelle Trageigenschaften der Auflageflächen der Lippen (5, 10) aufweist, die deren Quetschung zwischen den Lippen entgegenwirken.

3. Vorrichtung nach einem der vorstehenden Ansprüche, **dadurch gekennzeichnet, dass** bezogen auf eine Situation, in der die Vorrichtung zwischen den Lippen (95) eingeklemmt ist, und unter üblicher Berücksichtigung, dass die radiale Richtung (R) die Abstands- und Annäherungsrichtung der Lippen voneinander/zueinander und die axiale Richtung (A) die zur radialen Richtung orthogonale Ein- und Austrittsrichtung aus dem Mund bedeutet, die vorstehenden Wände (20, 22) sich in der radialen (R) oder im Wesentlichen radialen Richtung erstrecken, die Auflageflächen sich in der axialen (A) und/oder im Wesentlichen axialen Richtung und vorzugsweise nach Verbindungsabschnitten (5b, 10b) zu den vorstehenden Wänden erstrecken.

4. Vorrichtung nach einem der vorstehenden Ansprüche, **dadurch gekennzeichnet, dass** die Bissvorrichtung eine Stützwand zum Abstützen der dazwischen eingeklemmten Zahnbögen aufweist, wobei die Wand frei von Durchgängen für die Zunge ist.

5. Vorrichtung nach einem der vorstehenden Ansprüche, **dadurch gekennzeichnet, dass** die Auflageflächen (5, 10) bogenförmig oder im Wesentlichen bogenförmig sind, so dass sie in der Mitte der Bögen einen maximalen Abstand (D) zueinander aufweisen, wobei der Abstand (D) oder der minimale Abstand zwischen den Zentren der Bögen vorzugsweise größer als 1,5 cm ist.

6. Vorrichtung nach einem der vorstehenden Ansprüche, **dadurch gekennzeichnet, dass** sie vollständig aus elastischem Material besteht.

7. Vorrichtung nach einem der vorstehenden Ansprüche, **dadurch gekennzeichnet, dass** sie keine von den Auflageflächen (5, 10) vorstehenden Wände aufweist und dazu bestimmt ist, außerhalb des Mundes in einer Position gegenüber den vorstehenden Wänden (20, 22) positioniert zu werden, die dazu bestimmt sind, zwischen den Lippen und den Zahnbögen angeordnet zu werden.

8. Vorrichtung nach einem der vorstehenden Ansprüche, **dadurch gekennzeichnet, dass** sie einen Greifteil (30), vorzugsweise eine Stange, umfasst, der geeignet ist, aus dem Mund herauszuragen.

9. Vorrichtung nach einem der vorstehenden Ansprüche, **dadurch gekennzeichnet, dass** die Auflageflächen (5, 10) sich bogenförmig erstrecken und erste Abschnitte 5a, 10a, die sich in axialer und/oder im Wesentlichen axialer Richtung (A) erstrecken, und zweite Verbindungsabschnitte (5b, 10b) zu den vorstehenden Wänden (20, 22) umfassen, um eine Flächenkontinuität mit diesen zu ermöglichen, wobei:
- der erste Abschnitt (5a) der oberen Auflagefläche (5) eine Ausdehnung in axialer oder im Wesentlichen axialer Richtung hat, die von den Enden zur Mitte des entsprechenden Bogens hin dünner wird,
- während hingegen der zweite Teil (10a) der unteren Auflagefläche (10) eine Ausdehnung in axialer oder im Wesentlichen axialer Richtung hat, die von den Enden zur Mitte des entsprechenden Bogens hin dicker wird.

## Revendications

1. Dispositif oral myofonctionnel pour la rééducation myofonctionnelle, conçu pour être porté entre les lèvres d'un patient, qui comprend :
- une surface de support pour la lèvre supérieure (5),
- une surface de support pour la lèvre inférieure (10),
- une paroi (20) faisant saillie à partir de la surface de support supérieure (5) dans une position conçue pour être interposée entre la lèvre supérieure et l'arcade dentaire supérieure lorsque les surfaces de support sont serrées entre les lèvres ;
- une paroi (22) faisant saillie à partir de la surface de support inférieure (10) dans une position conçue pour être interposée entre la lèvre inférieure et l'arcade dentaire inférieure lorsque les surfaces de support sont serrées entre les lèvres ;
**caractérisé en ce que**
- les surfaces de support sont façonnées pour former une surface de contact annulaire continue (11) avec les lèvres ;
- les parois saillantes forment une collerette annulaire continue (19) autour de ladite surface de contact annulaire (11) ;
- la surface de contact annulaire (11) et la collerette annulaire (19) sont reliées l'une à l'autre d'une manière étanche à l'air le long de la totalité du périmètre annulaire, par exemple avec une continuité structurelle ;
- le dispositif oral myofonctionnel comprend une partie (16) qui obstrue le passage entre les lèvres placée à l'intérieur de la zone encerclée par la surface de contact annulaire (11) pour rendre étanche le passage d'air et de la langue, dans lequel
le dispositif oral myofonctionnel comprend un dispositif de type à morsure (202) joint aux surfaces de support de lèvre (11), le dispositif de morsure étant exempt de canaux d'aspiration de la langue à travers celui-ci
dans lequel ladite partie d'obstruction comprend au moins une partie de jonction avec ledit dispositif de morsure
et dans lequel ledit dispositif de morsure comprend au moins une rampe de support (260) pour porter la langue avec une inclinaison vers le point palatin pour reposer au-dessous de la langue et la soutenir.

2. Dispositif selon la revendication 1, **caractérisé en ce que** la partie d'obstruction (16) possède des caractéristiques de soutien structurel desdites surfaces de support de lèvre (5, 10) de façon à opposer leur écrasement entre les lèvres.

3. Dispositif selon l'une quelconque des revendications précédentes, **caractérisé en ce que**, en référence à une situation dans laquelle le dispositif est serré entre les lèvres (95) et en considérant classiquement que la direction radiale (R) correspond à la direction d'éloignement et de rapprochement des lèvres l'une de/vers l'autre et la direction axiale (A) correspond à la direction d'entrée et de sortie de la bouche orthogonale à la direction radiale, les parois saillantes (20, 22) s'étendent dans la direction radiale (R) ou sensiblement radiale, les surfaces de support s'étendent dans la direction axiale (A) et/ou sensiblement axiale et de préférence selon des parties de liaison (5b, 10b) avec les parois saillantes.

4. Dispositif selon l'une quelconque des revendications précédentes, **caractérisé en ce que** ledit dispositif de morsure comprend une paroi de support pour porter les arcades dentaires serrées entre elles, ladite paroi étant exempte de passages pour la langue à travers celle-ci.

5. Dispositif selon l'une quelconque des revendications précédentes, **caractérisé en ce que** les surfaces de support (5, 10) sont à géométrie arquée ou sensiblement arquée, de façon à présenter une distance maximale (D) entre elles au centre des arcades, où la distance (D), ou la distance minimale, entre les centres des arcades est de préférence supérieure à 1,5 cm.

6. Dispositif selon l'une quelconque des revendications précédentes, **caractérisé en ce qu'**il est entièrement constitué de matériau élastique.

7. Dispositif selon l'une quelconque des revendications précédentes, **caractérisé en ce qu'**il est exempt de parois faisant saillie à partir des surfaces de support (5, 10) et destiné à être positionné à l'extérieur de la bouche dans une position faisant face aux parois saillantes (20, 22) destinées à être interposées entre les lèvres et les arcades dentaires.

8. Dispositif selon l'une quelconque des revendications précédentes, **caractérisé en ce qu'**il comprend une partie de préhension (30), de préférence une tige, conçue pour faire saillie à l'extérieur de la bouche.

9. Dispositif selon l'une quelconque des revendications précédentes, **caractérisé en ce que** les surfaces de support (5, 10) s'étendent selon une arcade et comprennent des premières parties 5a et 10a qui s'étendent dans la direction axiale et/ou sensiblement axiale (A) et des secondes parties de liaison (5b, 10b) avec les parois saillantes (20, 22), de façon à permettre une continuité de surface avec celles-ci, où :
- la première partie (5a) de la surface de support supérieure (5) présente une extension dans la direction axiale ou sensiblement axiale qui devient plus mince, des extrémités vers le centre de l'arcade associée,
- au contraire, la seconde partie (10a) de la surface de support inférieure (10) présente une extension dans la direction axiale ou sensiblement axiale qui devient plus épaisse, des extrémités vers le centre de l'arcade associée.
